# DEMANDE DE BREVET EUROPEEN

(11) **EP 2 891 655 A1**
(43) Date de publication de la demande: **08.07.2015**
(21) Numéro de dépôt: 15150697.9
(22) Date de dépôt: 04.04.2012
(51) Int. Cl.: C07D 333/24, A61K 31/381, A61P 17/00

(54) **Anilides-alkylthioéthers comme inhibiteurs de l'ACAT pour le traitement de maladies dermatologiques**

(30) Priorité: 04.04.2011 FR 1152889
(62) Demande divisionnaire de: 12711677.0
(71) Demandeur: PIERRE FABRE MEDICAMENT, 92100 Boulogne-Billancourt (FR)
(72) Inventeur: Clerc, Thierry, 31320 Vigoulet Auzil (FR); Junquero, Didier, 81100 Castres (FR); Perez, Michel, 81100 Castres (FR); Autin, Jean-Marie, 81290 Viviers Les Montagnes (FR)
(74) Mandataire: Regimbeau

(57) **Abrégé**

La présente invention a pour objet des nouveaux alkylthioéthers de formule générale (I) pour la préparation d'un médicament destiné à prévenir ou à traiter un désordre dû à un dysfonctionnement des glandes sébacées chez l'homme ou l'animal. Les composés selon la présente invention exercent une forte inhibition de l'Acyl CoA:cholestérol O-Acyl transférase (ACAT). 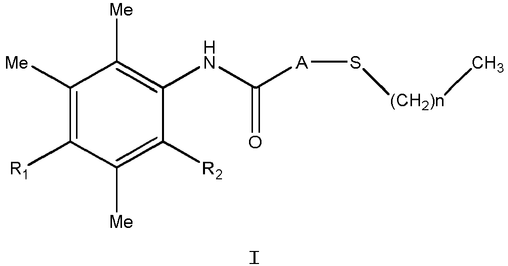 dans laquelle n représente un nombre entier de 5 à 13; R1 représente un groupe hydroxyle ou amino; R2 représente un hydrogène ou un radical méthyle; et A représente un groupement choisi parmi

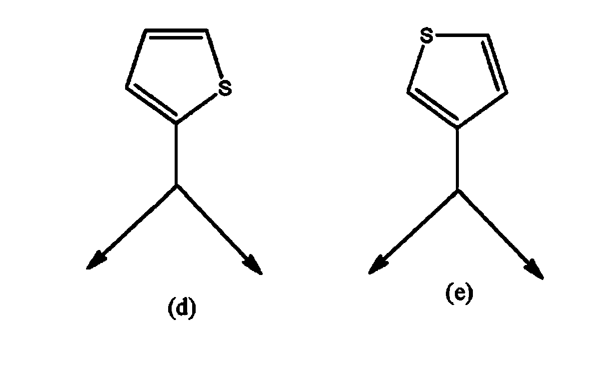

## Description

La présente invention se rapporte à de nouveaux alkylthioéthers pour la préparation d'un médicament destiné à prévenir ou à traiter un désordre dû à un dysfonctionnement des glandes sébacées chez l'homme ou l'animal, comme par exemple l'acné et/ou tout état ou pathologie lié à une surproduction de sébum, comme par exemple la dermite séborrhéique, la peau grasse ou le cuir chevelu gras, ou toute complication qui en sont issues. Par ailleurs ledit médicament peut être utilisé pour diminuer la charge lipidique (triacylglycerol et cholesteryl-esters) des adipocytes situés dans les couches profondes de la peau comme l'hypoderme. L'activité d'inhibition de l'estérification du cholestérol au niveau des adipocytes de l'hypoderme peut être obtenue après application par voie topique des inhibiteurs de l'ACAT exemplifiés ci-après. Enfin, les composés décrits dans la présente invention peuvent être utilisés pour activer les processus de cicatrisation apparaissant lors de foyers inflammatoires, quel que soit l'origine de l'inflammation. On entend par médicament toute préparation galénique permettant une bonne biodisponibilité des nouveaux dérivés vers la cible enzymatique définie ci-après : topique, formes orales ou toute autre formulation galénique judicieusement choisie. Les composés selon la présente invention exercent une forte inhibition de l'Acyl CoA:cholestérol O-Acyl transferase (ACAT), encore appelée Sterol - O- Acyl transferase (SOAT).

Les inhibiteurs de l'ACAT ont initialement été développés dans le but de contrôler l'homéostasie du cholestérol hépatique ou intestinal dans le but d'en limiter la circulation. Les inhibiteurs de la synthèse du cholestérol ont donné lieu à une classe de médicament nommée statine, dont la prise permet de faire diminuer la cholestérolémie. Ces traitements permettent de limiter l'apparition de la plaque d'athérome, et par voie de conséquence, les obstructions coronaires et la mortalité qui en découle (Baigent C. et coll., Lancet 2010). D'autres classes de produits ont été développées visant d'autres cibles enzymatiques dans cette indication, comme la cholestérol 7 alpha hydroxylase ou encore l'ACAT (Nissen S.E., et coll., NEJM 2006). Cliniquement l'inhibition de l'ACAT n'a pas donné de résultats probants en termes d'efficacité dans l'indication visée. Cependant, un des effets secondaires majeurs de l'inhibition de l'ACAT est l'atrophie des glandes sébacées, comme cela est observé chez le primate traité chroniquement par le composé PD 132301-2 par Reindel J.F. et coll., (Tox. Pathol., 1994). Ainsi il devient clair pour le spécialiste, que toute inhibition adéquate de l'ACAT pourrait conduire à résoudre les problèmes posés par la surproduction de sébum par les glandes sébacées. Cette inhibition peut être obtenue par l'administration de composés d'intérêt par voie orale ou bien par toute autre voie d'administration, comme les gels, onguents, émulsions, crèmes ou tout système transdermiques permettant un bon ciblage de l'ACAT, enzyme active au niveau des glandes pilosébacées. La glande sébacée est considérée comme un organe endocrine. Outre sa fonction de production du sébum, elle intervient dans la régulation de la stéroïdogénèse (Thiboutot et coll., J. Invest. Dermatol, 2003). La séborrhée correspond à une production excessive de sébum par les glandes sébacées. La majorité des lipides de la surface de l'épiderme provient des glandes sébacées, alors que les lipides produits par les autres sites de l'épiderme sont minimes en quantité (Greene R.S., et coll, J Invest Dermatol, 1970). Le sébum est composé de 57% de triglycérides, diglycérides, acides gras libres, de 26% de cires, 15% de squalène et de seulement 2% de cholestérol. C'est l'objet de la présente invention de montrer que les composés exemplifiés ci-après bloquent l'estérification du cholestérol rendant impossible le rôle stabilisateur du cholestérol estérifié au sein de la sécrétion lipidique de la glande sébacée. Le cholestérol estérifié joue le rôle de stabilisant dans la physico-chimie des structures lipidiques lamellaires puis vésiculaires qui composent la sécrétion des glandes sébacées. De par sa localisation au niveau du réticulum endoplasmique, l'ACAT-1 a pour rôle le stockage du cholestérol sous forme estérifiée. L'ACAT-1 est présente dans de nombreux tissus, mais plus fortement exprimée au niveau des macrophages des glandes surrénales et sébacées; contrairement à l'ACAT-2 qui est principalement exprimée au niveau de l'intestin grêle et du foie et dont la distribution tissulaire est moins ubiquitaire que l'isoforme 1. L'ACAT-2 procure le cholestérol estérifié qui structure les lipoprotéines et chylomicrons, alors que l'ACAT-1 fourni le cholestérol estérifié cytoplasmique des glandes surrénales et sébacées (Chang T.Y., Annu Rev. Biochem, 1997). La sécrétion lipidique des glandes sébacées utilise cet agent structurant, en plus des autres lipides cités précédemment.

Par ailleurs, l'inhibition de l'ACAT-1 peut être d'intérêt dans l'optimisation des processus de cicatrisation lors des phénomènes inflammatoires. En effet il est rapporté dans la littérature que certains inhibiteurs comme le produit K-604 très sélectif de l'isoforme ACAT-1 (moins actif sur l'isoforme ACAT-2) pouvaient induire une augmentation de procollagène de type 1. Cette augmentation est reportée autant sur le taux de protéine que sur celui du mRNA codant pour le procollagène de type 1. Par comparaison les composés inhibiteurs moins sélectifs de l'isoforme ACAT-1, comme le pactimibe, n'ont pas cette propriété (Yoshinaka et coll., Atherosclerosis, 2010). La cicatrisation est une réorganisation conjonctive du foyer inflammatoire. La réaction inflammatoire, induit la formation d'un granulome qui se transforme progressivement en un blastème de régénération. Il est constitué de fibroblastes qui sécrètent, entre autres, les composants de la matrice extracellulaire : glycosaminoglycanes, fibronectine et collagène. Juste après la détersion, le bourgeon charnu est un tissu conjonctif jeune, oedémateux, pauvre en collagène et en vaisseaux, riche en fibroblastes et en cellules inflammatoires. Progressivement, ce tissu s'appauvrit en cellules inflammatoires et s'enrichit en collagène et en vaisseaux. Ainsi il existe une phase importante au cours de laquelle la synthèse de collagène est primordiale. Les composés inhibiteurs de l'ACAT-1 permettent d'accélérer ce processus en stimulant la production de collagène à partir du procollagène de type 1 lors des situations inflammatoires. Ainsi, les composés inhibiteurs de l'ACAT-1 devraient améliorer la cicatrisation cutanée, notamment dans le traitement des brûlures de toutes origines, ainsi que dans le traitement des ulcères de la peau.

Enfin il existe un intérêt évident du blocage de l'ACAT-1 afin de limiter la charge lipidique et donc le stockage des lipides au niveau intra-adipocytaire. Ainsi la diminution du stockage lipidique obtenu par l'inhibition de l'enzyme ACAT-1 permet indirectement de réduire le nombre de vésicules lipidiques intracellulaires et donc l'hyperplasie adipocytaire. L'intérêt des inhibiteurs de l'ACAT-1 est de proposer une solution médicamenteuse par voie orale ou par voie topique qui contrôle le stockage des lipides au niveau sous-cutané.

Les composés de la présente invention ont la capacité d'inhiber sélectivement l'ACAT-1 ce qui devrait se traduire, chez l'homme, par une action bénéfique pour le traitement de maladies telles que l'obésité, les dyslipidémies, l'hypertriglycéridémie, l'hypercholestérolémie, le syndrome métabolique, l'athérosclérose, la stéatose hépatique, les risques cardiovasculaires; pour le traitement et/ou prévention des désordres lipidiques de la peau, comme l'acné, le psoriasis, la rosacée, la dermite séborrhéique, la séborrhée, l'eczéma, mais aussi la peau grasse ou le cuir chevelu gras ; pour le traitement des plaies chroniques ; pour le traitement de la surcharge lipidique sous-cutanée omentale observable chez les individus obèses ou en surpoids.

La présente invention concerne également les combinaisons entre les composés décrits et d'autres agents utilisés dans le traitement ou la prévention des maladies liées aux désordres lipidiques de la peau tel que les rétinoïdes, les antibiotiques, les antibactériens, les anti-androgènes, les antifongiques, les corticoïdes.

Ces composés se distinguent de l'art antérieur par leur structure chimique différente et leur propriété biologique remarquable.

Les composés de la présente invention sont de formule générale (I) : 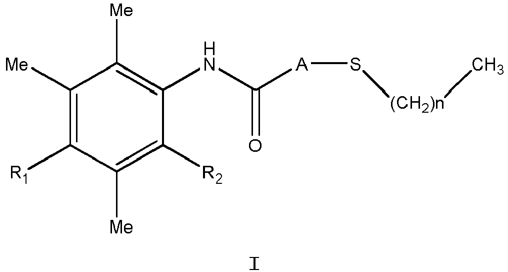 dans laquelle :
- R₁ représente un groupe hydroxyle ou amino
- R₂ représente un hydrogène ou un radical méthyle
- A représente un groupement choisi parmi : 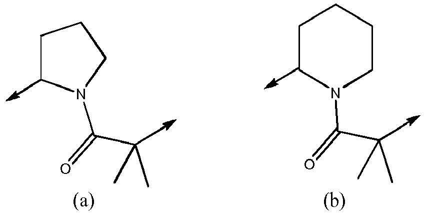 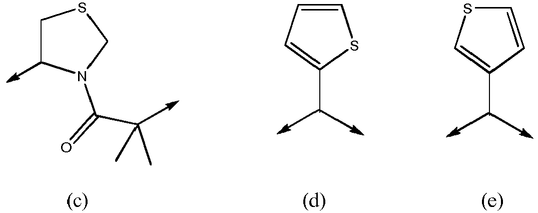
- n représente un nombre entier de 5 à 13,
   ainsi que leurs sels et solvates acceptables pour l'usage thérapeutique.

Les flèches représentent les points d'attachement à la formule générale (I).

Dans les définitions qui précèdent :

Toutes les combinaisons de substituants ou de variables sont possibles dans la mesure où elles conduisent à des composés stables.

Les sels acceptables pour l'usage thérapeutique des composés de la présente invention comprennent les sels non toxiques conventionnels des composés de l'invention tels que ceux formés à partir d'acides organiques ou inorganiques ou de bases organiques ou inorganiques. A titre d'exemple on peut citer les sels dérivés d'acides inorganiques comme les acides chlorhydrique, bromhydrique, phosphorique, sulfurique, et ceux dérivés d'acides organiques comme les acides acétique, trifluoroacétique, propionique, succinique, fumarique, malique, tartarique, citrique, ascorbique, maléique, glutamique, benzoïque, salicylique, toluenesulfonique, méthanesulfonique, stéarique, lactique. A titre d'exemple on peut citer les sels dérivés de bases inorganiques comme la soude, la potasse ou l'hydroxyde de calcium et les sels dérivés de bases organiques comme la lysine ou l'arginine.

Ces sels peuvent être synthétisés à partir des composés de l'invention contenant une partie basique ou acide et les acides ou bases correspondants selon les méthodes chimiques conventionnelles.

Les solvates acceptables pour l'usage thérapeutique des composés de la présente invention comprennent les solvates conventionnels tels que ceux formés lors de la dernière étape de préparation des composés de l'invention du fait de la présence de solvants. A titre d'exemple on peut citer les solvates dus à la présence d'eau ou d'éthanol.

Tous les stéréoisomères y compris tous les isomères optiques des composés de formule générale (I) font également partie de la présente invention ainsi que leur mélange sous forme racémique.

Parmi les composés de formule générale (I) faisant partie de la présente invention, une classe de composés particulièrement appréciée correspond aux composés de formule générale (I) dans laquelle A représente le groupement (a), R₁ représente un hydroxyle (OH) et R₂ représente un hydrogène.

Parmi les composés de formule générale (I) faisant partie de la présente invention, une classe de composés particulièrement appréciée correspond aux composés de formule générale (I) dans laquelle A représente le groupement (b), R₁ représente un hydroxyle (OH) et R₂ représente un hydrogène.

Parmi les composés de formule générale (I) faisant partie de la présente invention, une classe de composés particulièrement appréciée correspond aux composés de formule générale (I) dans laquelle A représente le groupement (c), R₁ représente un hydroxyle (OH) et R₂ représente un hydrogène.

Parmi les composés de formule générale (I) faisant partie de la présente invention, une classe de composés particulièrement appréciée correspond aux composés de formule générale (I) dans laquelle A représente le groupement (d), R₁ représente une amine (NH₂) et R₂ représente un méthyle.

Parmi les composés de formule générale (I) faisant partie de la présente invention, une classe de composés particulièrement appréciée correspond aux composés de formule générale (I) dans laquelle A représente le groupement (e), R₁ représente un hydroxyle (OH) ou une amine (NH₂) et R₂ représente un hydrogène ou un méthyle.

La présente invention concerne également la préparation des composés de formule générale (I) par les procédés généraux décrits dans les schémas synthétiques suivants complétés, le cas échéant, de toutes les manipulations standards décrites dans la littérature ou bien connues de l'homme du métier ou bien encore exemplifiées dans la partie expérimentale.

Les compositions pharmaceutiques comprenant les composés selon l'invention en association avec tout excipient approprié sont également envisagées. Le terme excipient approprié se réfère à des entités moléculaires et des compositions qui ne produisent aucun effet adverse, allergique ou autre réaction indésirable quand elles sont administrées à un animal ou un humain. Quand utilisé ici, le terme «excipient approprié» inclut tout diluant, adjuvant ou excipient, tels que des agents préservatifs, des agents de remplissage, des agents désintégrants, mouillants, émulsifiants, dispersants, antibactériens ou antifongiques, ou bien encore des agents qui permettraient de retarder l'absorption et la résorption intestinale et digestive. L'utilisation de ces milieux ou vecteurs est bien connue de l'homme de l'art.

### Description des synthèses

Les composés de la présente invention peuvent être synthétisés en utilisant les voies de synthèse décrites ci-dessous ou en utilisant des méthodes de synthèse connues de l'homme du métier.

### Méthode 1

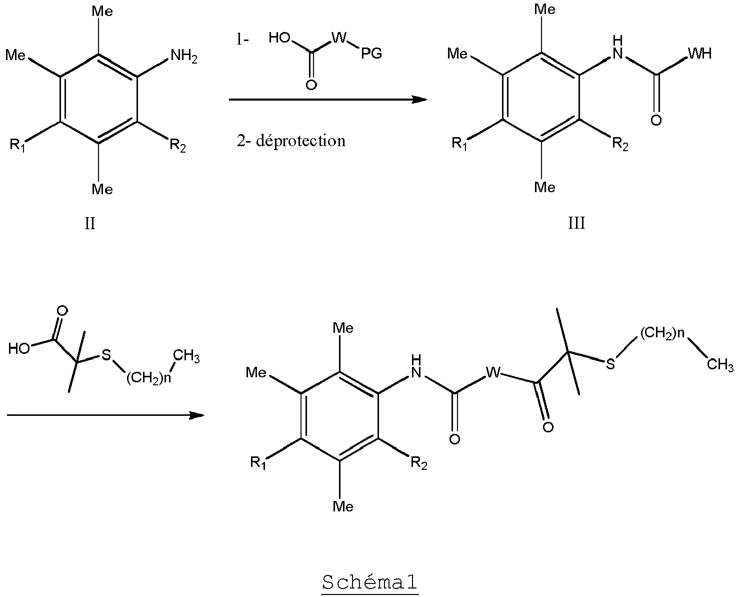

La synthèse des composés de formule générale I est caractérisée (schéma 1) en ce que l'on condense un dérivé de formule générale II 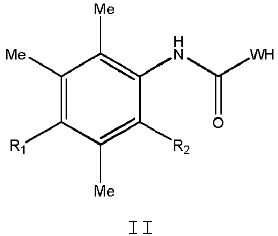 dans laquelle R₁ et R₂ représentent les groupements tels que décrits précédemment dans la formule I et W peut être la pyrrolidine, la pipéridine ou la thiazolidine avec la fonction C=O branchée respectivement en C2 pour les 2 cycles azotés et en C4 pour le dérivé soufré, avec un dérivé de formule générale III 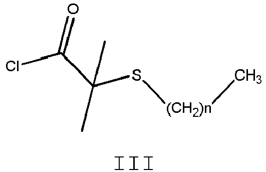 dans laquelle n est tel que décrit précédemment dans la formule I. Cette réaction peut être effectuée en présence d'une base organique ou inorganique telle que par exemple Et₃N, iPr₂NEt, NaH, pyridine, CseCO₃, K₂CO₃, NaOH, KOH dans un solvant anhydre polaire tel que le THF, le DMF, le DMSO, l'acétone à une température comprise entre - 20° et 140°C.

### Méthode 2

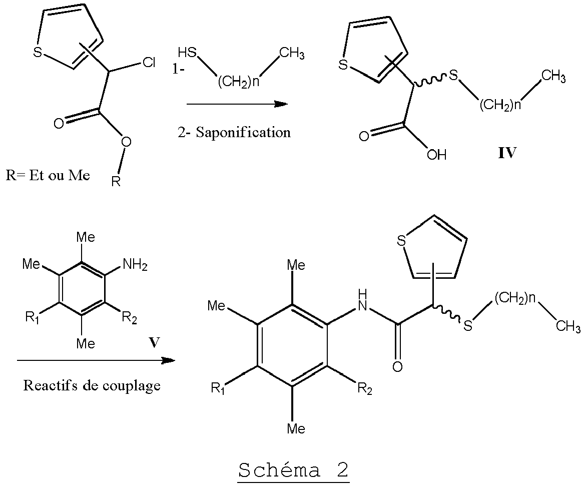

Cette méthode de synthèse des composés de formule générale I (schéma 2), est caractérisée en ce que l'on condense un dérivé de formule générale IV obtenu en 2 étapes à partir du méthyl ou éthyl 2-chloro-(thiophen-2-yl)acétate (commerciaux). La réaction consiste à faire réagir ces réactifs avec un thiol de formule HS(CHₑ)ₙCH₃ avec n défini comme dans la formule générale dans un solvant tel que cette réaction peut être réalisée en présence d'une base organique ou inorganique telle que par exemple Et₃N, iPr₂NEt, NaH, pyridine, Cs₂CO₃, K₂CO₃ dans un solvant anhydre polaire tel que le THF, le DMF, le DMSO, l'acétone à une température comprise entre - 20° et 140°C, en présence ou non d'un sel comme catalyseur et qui peut être KI, Bu₄NI, LiI, AgBF₄, AgClO₄, Ag₂CO₃, KF, Bu₄NF ou CsF. 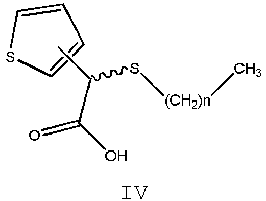 dans lequel n est tel que décrit précédemment dans la formule I et le positionnement de la chaîne soufrée peut être en position 2 ou 3 du thiophène, avec un composé de formule générale V 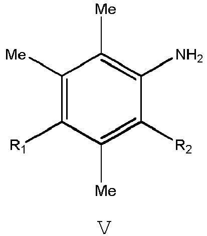 dans lequel R₁ et R₂ représentent les groupements tels que décrits précédemment dans la formule I.

Cette réaction peut être réalisée par les méthodes et techniques bien connues de l'homme de l'art. Une méthode particulièrement appréciée consiste à condenser ces 2 entités en présence d'une amine tertiaire telle que la triéthylamine et de N,N'-dicyclohexylcarbodiimide dans le dichlorométhane. Ou encore, à titre d'exemple, en présence de 1-(3-diméthylaminopropyl)-3-éthyl-carbodiimide (EDC), de 3-hydroxy-1,2,3-benzotriazin-4(3H)-one, d'une amine tertiaire telle que la diisopropyléthylamine, dans un solvant aprotique polaire tel que le dichlorométhane ou le DMF, à une température comprise entre - 15°C et 50°C. Ou encore, à titre d'exemple, en utilisant le benzotriazol-1-yloxy-tris(diméthylamino) phosphonium hexafluorophosphate (BOP) en présence de 1-hydroxybenzotriazole, d'une amine tertiaire telle que la diisopropyléthylamine, dans un solvant polaire tel que le DMF, le CH₂Cl₂ ou le DMSO à une température comprise entre 10° et 50°C. Une autre méthode particulièrement appréciée consiste à transformer l'acide carboxylique en chlorure d'acide par réaction avec le chlorure d'oxalyle ou le chlorure de thionyle en l'absence ou en présence d'une base telle que la pyridine ou la triéthylamine sans ou avec un solvant tel que le toluène ou le dichlorométhane à une température comprise entre 20 et 100°C. Ce chlorure d'acide peut ensuite réagir avec l'amine HNR₇R₈ en présence d'une base telle que la pyridine ou la triéthylamine dans un solvant tel que le dichlorométhane à une température comprise entre 0 et 100°C.

Les matières premières utilisées dans les procédés décrits précédemment sont commercialisées ou aisément accessibles à l'homme du métier selon des procédés décrits dans la littérature.

Les sels acceptables pour l'usage thérapeutique des composés de la présente invention comprennent les sels non toxiques conventionnels des composés de l'invention tels que ceux formés à partir d'acides organiques ou inorganiques ou de bases organiques ou inorganiques. A titre d'exemple on peut citer les sels dérivés d'acides inorganiques comme les acides chlorhydrique, bromhydrique, phosphorique, sulfurique, et ceux dérivés d'acides organiques comme les acides acétique, trifluoroacétique, propionique, succinique, fumarique, malique, tartarique, citrique, ascorbique, maléique, glutamique, benzoïque, salicylique, toluenesulfonique, méthanesulfonique, stéarique, lactique. Ces sels peuvent être synthétisés à partir des composés de l'invention contenant une partie basique et les acides correspondants selon les méthodes chimiques conventionnelles.

Les solvates acceptables pour l'usage thérapeutique des composés de la présente invention comprennent les solvates conventionnels tels que ceux formés lors de la dernière étape de préparation des composés de l'invention du fait de la présence de solvants. A titre d'exemple on peut citer les solvates dus à la présence d'eau ou d'éthanol.

Tous les stéréoisomères y compris tous les isomères optiques des composés de formule générale (I) font également partie de la présente invention ainsi que leur mélange sous forme racémique.

Les exemples suivants illustrent l'invention sans en limiter la portée.

Les analyses élémentaires et les spectres de masse et RMN confirment les structures des composés. Les composés intermédiaires et finaux peuvent être, si on le désire, purifiés suivant une ou plusieurs méthodes de purification choisies parmi, l'extraction, la filtration, la chromatographie sur gel de silice, l'HPLC préparative sur phase normale ou inverse, la cristallisation.

### Description des intermédiaires

### Intermédiaires 1

### a) acide 2-(dodécylthio)-2-méthylpropanoïque (1a)

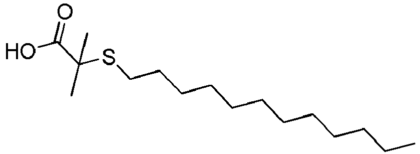

Le dodécane-1-thiol (8,63 g, 0,036 mol) est additionné à de l'hydrure de sodium à 45% dans la paraffine (1,82g, 0,036 mol) à température ambiante. Le mélange est chauffé à 120°C pendant 1h. Le milieu réactionnel ramené à température ambiante est solubilisé par 36mL d'éthanol, l'éthyl-2-bromo-isobutyrate (5,28mL, 0,036 mol), est additionné au milieu qui est chauffé à reflux pendant 4h. Après filtration et concentration à sec, 11,4 g d'huile sont obtenus. CCM gel de silice 60 F 254 Merck, Hexane-AcOEt : 70-30, Rf = 0,62. Cette huile est mise en présence d'hydroxyde de potassium (4,04g, 0,036 mol) dans 36mL d'éthanol et 24mL d'eau. Le milieu est chauffé à reflux pendant 4h. Après refroidissement, le milieu est acidifié par addition d'acide chlorhydrique 6N, après ajout de glace et d'eau, le précipité formé est filtré, lavé à l'hexane. L'intermédiaire la est obtenu sous forme de poudre blanche (10,05g, rendement : 96%).
Point de fusion : 69°C.
CCM gel de silice 60 F 254 Merck, Hexane-AcOEt : 70-30, Rf = 0,62.

### b) acide 2-(décylthio)-2-méthylpropanoïque (1b)

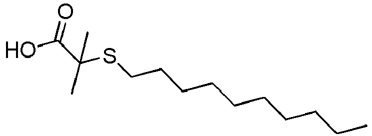

La synthèse de l'intermédiaire 1b est réalisée à partir du décane-1-thiol suivant le mode opératoire décrit pour la synthèse de 1a.

### Intermédiaires 2

### a)N-(4-hydroxy-2,3,5-triméthylphényl)pipéridine-2-carboxamide (2a)

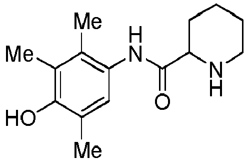

L'acide DL-pipécolinique (5,17g, 0,04 mol) est solubilisé dans 44mL de soude 1N, 30mL de tert-butanol sont additionnés, puis le di-tert-butyl dicarbonate (8,73g, 0,044 mol) est ajouté par fractions à température ambiante. Le milieu est agité pendant 24h, puis dilué à l'eau, acidifié à pH2 par addition d'acide sulfurique. Le milieu réactionnel est ensuite extrait à l'acétate d'éthyle, lavé deux fois à l'eau et les phases organiques sont séchées sur MgSO₄. Après filtration et concentration à sec, le précipité obtenu est repris à l'hexane, puis filtré. 5,18g de poudre blanche sont isolés (rendement : 56%). Point de fusion : 132°C. CCM gel de silice 60 F 254 Merck, CH₂Cl₂-MeOH : 90-10, Rf = 0,51. L'intermédiaire obtenu (4,17g, 0,0182 mol) est mis en solution à température ambiante dans 45mL de dichorométhane en présence de triéthylamine (2,5mL, 0,0182 mol). Après refroidissement à 0°C, le chloroformate d'éthyle (1,74mL, 0,0182 mol) est additionné goutte à goutte, le mélange est agité 20min. L'addition sous azote de triéthylamine (2,6mL, 0,0182 mol) à une suspension de chlorhydrate de 4-amino-2,3,6-triméthylphénol (2,84g, 0,0151 mol) dans 80mL de dichlorométhane conduit à un mélange qui est glacé et ajouté lentement à la première solution. Le milieu est ensuite laissé sous agitation à température ambiante pendant 18h. Le milieu réactionnel est ensuite dilué dans l'eau, extrait à l'acétate d'éthyle, lavé deux fois à l'eau et avec une solution aqueuse saturée en NaCl et les phases organiques sont séchées sur MgSO₄. Après filtration et concentration à sec, le précipité obtenu est repris à l'éther, puis filtré. 4,15g de poudre blanche sont isolés (rendement : 76%). Point de fusion : 132°C. CCM gel de silice 60 F 254 Merck, Hexane-AcOEt : 50-50, Rf = 0,55. Cette poudre est mise en suspension dans 42mL de dichlorométhane en présence d'acide trifluoroacétique (11,6mL, 0,066 mol). Après agitation pendant 1h30 à température ambiante, le milieu réactionnel est amené à pH8 par addition d'une solution aqueuse saturée en NaHCO₃, puis il est extrait à l'acétate d'éthyle, la phase organique est séchée sur MgSO₄. Après filtration et concentration à sec, le précipité obtenu est repris à l'hexane, puis filtré. 1,01g d'intermédiaire 2a sous forme d'une poudre blanche sont isolés (rendement : 34%).
Point de fusion : 190°C.
CCM gel de silice 60 F 254 Merck, CH₂Cl₂-MeOH : 85-15, Rf = 0,20.

### b)N-(4-hydroxy-2,3,5-triméthylphényl)pyrrolidine-2-carboxamide (2b)

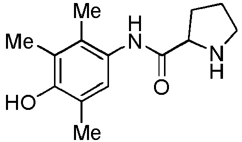

La synthèse de l'intermédiaire 2b est réalisée à partir de la DL-proline suivant le mode opératoire décrit pour la synthèse de 2a. (poudre blanche, rendement 36% sur les 3 étapes).
Point de fusion : 223°C.
CCM gel de silice 60 F 254 Merck, CH₂Cl₂-MeOH : 85-15, Rf = 0,30.

### c)(S)-N-(4-hydroxy-2,3,5-triméthylphényl)pyrrolidine-2-carboxamide (2c)

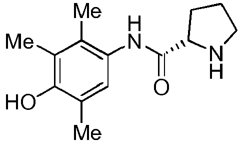

La synthèse de l'intermédiaire 2c est réalisée à partir de la L-proline suivant le mode opératoire décrit pour la synthèse de 2a. (poudre blanche, rendement 22% sur les 3 étapes).
Point de fusion : 193°C.
CCM gel de silice 60 F 254 Merck, CH₂Cl₂-MeOH : 85-15, Rf = 0,20.

### d)(R)-N-(4-hydroxy-2,3,5-triméthylphényl)thiazolidine-4-carboxamide (2d)

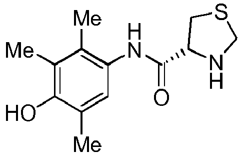

La synthèse de l'intermédiaire 2d est réalisée à partir de l'acide L-4-thiazolidine-carboxylique suivant le mode opératoire décrit pour la synthèse de 2a. (poudre blanche, rendement 52% sur les 3 étapes).
Point de fusion : 237°C.
CCM gel de silice 60 F 254 Merck, Hexane-AcOEt : 50-50, Rf = 0,47.

### Intermédiaires 3

### a)acide 2-(dodécylthio)-2-(thiophén-3-yl)acétique (3a)

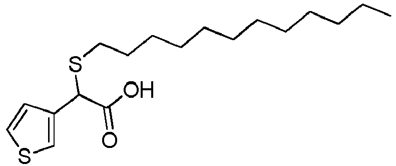

L'acide 3-thiényl acétique (14,36 g, 0,08 mol) est mis en suspension dans 80mL de dichlorométhane à température ambiante. Sont additionnés au milieu, 8mL de méthanol et la N,N'-dicyclohexylcarbodiimide (16,48g, 0,08 mol). Le mélange est agité pendant 3h, puis filtré sur büchner. Le filtrat est concentré puis repris à l'acétate d'éthyle, lavé avec une solution aqueuse saturée en NaHCO₃, à l'eau et avec une solution aqueuse saturée en NaCl et les phases organiques sont séchées sur MgSO₄. Après filtration et concentration à sec, 11,81 g d'huile sont obtenus (rendement : 95%). CCM gel de silice 60 F 254 Merck, Ether de Pétrole-AcOEt : 90-10, Rf = 0,48. Cette huile est mise en présence de N,N-diméthylformamide diméthylacétal (21,2mL, 0,15 mol) dans 70mL de diméthylformamide. Le milieu est chauffé à 100°C pendant 2h. Après refroidissement, le milieu est dilué à l'eau, extrait à l'acétate d'éthyle, la phase organique est lavée à l'eau puis avec solution aqueuse saturée en NaCl. La phase organique est séchée sur MgSO₄. Après filtration et concentration à sec, 11,34g d'une huile jaune sont isolés (rendement : 71%). CCM gel de silice 60 F 254 Merck, Ether de pétrole-AcOEt : 80-20, Rf = 0,49. Une solution de périodate de sodium (34g, 0,159 mol) dans 220mL d'eau est additionnée à l'huile précédemment obtenue solubilisée dans 220mL de tétrahydrofurane. Le milieu est agité 2h30 à température ambiante. Après concentration, le résidu est repris à l'acétate d'éthyle, la phase organique est lavée à l'eau puis avec solution aqueuse saturée en NaCl, puis séchée sur MgSO₄. Après filtration et concentration à sec, l'huile obtenue est purifiée par chromatographie flash sur silice (Ether de Pétrole-AcOEt 80-20). 7,5g d'une poudre jaune sont isolés (rendement : 82%). CCM gel de silice 60 F 254 Merck, Ether de pétrole-AcOEt : 80-20, Rf = 0,56. Cette poudre est mise en solution dans 185mL de méthanol. Après refroidissement à 0°C, une solution de borohydrure de sodium (0,83g, 0,022mol) dans 8mL d'eau est additionnée goutte à goutte en maintenant la température du milieu inférieure à 2°C. Après agitation pendant 30min, le milieu toujours à 0°C est neutralisé par addition de 5mL d'acide acétique en solution dans 90mL d'eau. Après 10min d'agitation, le milieu est concentré, le résidu est repris à l'acétate d'éthyle, la phase organique est lavée à l'eau puis avec une solution aqueuse saturée en NaCl, puis séchée sur MgSO₄. Après filtration et concentration à sec, 5,1g d'une huile incolore sont isolés (rendement : 68%). CCM gel de silice 60 F 254 Merck, Ether de pétrole-AcOEt :70-30, Rf = 0,31. L'huile obtenue est solubilisée dans 120mL de toluène en présence de pyridine (4,85mL, 0,06 mol). Après refroidissement à 0°C, le chlorure de thionyle (4,37mL, 0,06 mol) est additionné lentement. Après retour à température ambiante, le milieu est dilué abondamment à l'eau et extrait à l'acétate d'éthyle, la phase organique est lavée à l'eau puis avec solution aqueuse saturée en NaCl, puis séchée sur MgSO₄. Après filtration et concentration à sec, l'huile obtenue est purifiée par chromatographie flash sur silice (Ether de Pétrole-AcOEt 90-10). 4,7g d'une huile jaune sont isolés (rendement : 82%). CCM gel de silice 60 F 254 Merck, Ether de pétrole-AcOEt : 90-10, Rf = 0,36. A une solution de 1-dodécanethiol (6,69mL, 0,028 mol) dans 250mL de tétrahydrofurane, est ajouté le tert-butylate de potassium (3,14g, 0,028 mol). Après une heure d'agitation à température ambiante, est ajoutée une solution de l'huile précédemment obtenue solubilisée dans 10mL de tétrahydrofurane. Le milieu est agité 5h à température ambiante. Après concentration, le résidu est repris à l'eau et extrait à l'acétate d'éthyle, la phase organique est lavée à l'eau puis avec solution aqueuse saturée en NaCl, puis séchée sur MgSO₄. Après filtration et concentration à sec, l'huile obtenue est purifiée par chromatographie flash sur silice (Ether de Pétrole-AcOEt 98-2). 6,58g d'une huile incolore sont isolés (rendement : 77%). CCM gel de silice 60 F 254 Merck, Ether de pétrole-AcOEt : 98-2, Rf = 0,25. L'ester précédemment isolé est mis en suspension dans 120mL d'éthanol en présence d'une solution de soude 1N (36mL, 0,036 mol ). Après 5h d'agitation à température ambiante, l'éthanol est évaporé, le milieu est amené à pH acide par addition d'une solution aqueuse d'acide chlorhydrique 1N, puis il est extrait à l'acétate d'éthyle, la phase organique est séchée sur MgSO₄. Après filtration et concentration à sec, l'huile obtenue est glacée et reprise à l'éther de pétrole, le précipité formé est isolé par filtration et lavé à l'éther de pétrole. 4,74g d'intermédiaire 3a sous forme d'une poudre blanche sont isolés (rendement : 77%).
Point de fusion : 54°C.
CCM gel de silice 60 F 254 Merck, CH₂Cl₂-MeOH : 90-10, Rf = 0,59.

### b) acide 2-(dodécylthio)-2-(thiophén-2-yl)acétique (3b)

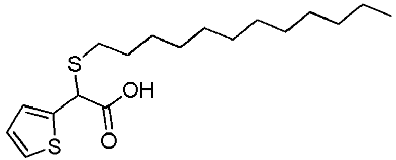

L'éthyl 2-oxo-2-(thiophén-2-yl)acétate (10g, 0,0543 mol) est mis en solution dans 220mL d'éthanol. Après refroidissement à 0°C, une solution de borohydrure de sodium (1,03g, 0,0271mol) dans 11mL d'eau est additionnée goutte à goutte en maintenant la température du milieu inférieure à 2°C. Après agitation pendant 5min, le milieu toujours à 0°C est neutralisé par addition de 6,2mL d'acide acétique en solution dans 110mL d'eau. Après 10min d'agitation, le milieu est concentré, le résidu obtenu est repris à l'acétate d'éthyle, la phase organique est lavée à l'eau, puis séchée sur MgSO₄. Après filtration et concentration à sec, 7,03g d'une huile jaune sont isolés (rendement : 70%). CCM gel de silice 60 F 254 Merck, Ether de pétrole-AcOEt : 70-30, Rf = 0,44. 6,53g de l'huile obtenue sont solubilisés dans 130mL de toluène en présence de pyridine (5,68mL, 0,0703 mol). Après refroidissement à 0°C, le chlorure de thionyle (5,11mL, 0,0703 mol) est additionné lentement. Après retour à température ambiante, le milieu est dilué abondamment à l'eau et extrait à l'acétate d'éthyle, la phase organique est lavée à l'eau puis avec solution aqueuse saturée en NaCl, puis séchée sur MgSO₄. Après filtration et concentration à sec, l'huile obtenue est purifiée par chromatographie flash sur silice (Ether de Pétrole-AcOEt 95-5). 4,06g d'une huile jaune sont isolés (rendement : 57%). CCM gel de silice 60 F 254 Merck, Ether de pétrole-AcOEt : 95-5, Rf = 0,32. A une solution de 1-dodécanethiol (5,68mL, 0,0237 mol) dans 200mL de tétrahydrofurane, est ajouté le tert-butylate de potassium (2,66g, 0,0237 mol). Après une heure d'agitation à température ambiante, est ajoutée une solution de l'huile précédemment obtenue solubilisée dans 25mL de tétrahydrofurane. Le milieu est agité 4h à température ambiante. Après concentration, le résidu est repris à l'eau et extrait à l'acétate d'éthyle, la phase organique est lavée à l'eau puis avec solution aqueuse saturée en NaCl, puis séchée sur MgSO₄. Après filtration et concentration à sec, l'huile obtenue est purifiée par chromatographie flash sur silice (Ether de Pétrole-AcOEt 99-1). 5,05g d'une huile jaune sont isolés (rendement : 69%). CCM gel de silice 60 F 254 Merck, Ether de pétrole-AcOEt :99-1, Rf = 0,51. L'ester précédemment isolé est mis en suspension dans 90mL d'éthanol en présence d'une solution de soude 1N (27,1mL, 0,0271 mol). Après 5h d'agitation à température ambiante, l'éthanol est évaporé, le milieu est amené à pH acide par addition d'une solution aqueuse d'acide chlorhydrique 1N, puis il est extrait à l'acétate d'éthyle, la phase organique est séchée sur MgSO₄. Après filtration et concentration à sec, l'huile obtenue est purifiée par chromatographie flash sur silice (CH₂Cl₂-MeOH : 90-10). 4,11g d'intermédiaire 3b sous forme d'une poudre orange sont isolés (rendement : 88%).
CCM gel de silice 60 F 254 Merck, CH₂Cl₂-MeOH : 90-10, Rf = 0,50.

### EXEMPLES

### Exemple 1: 1-(2-(dodécylthio)-2-méthylpropanoyl)-N-(4-hydroxy-2,3,5-triméthyl-phényl)pipéridine-2-carboxamide

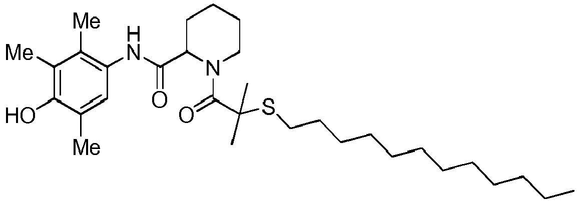

Le composé 1 est préparé selon la méthode de synthèse 1: 1,19g (3,81 mmoles) de dérivé la et 1,2mL de chlorure de thionyle (1,64 mmoles) sont placés dans 19mL de toluène. Le mélange est chauffé à reflux pendant 5h, puis le milieu réactionnel est concentré à sec, repris au toluène et évaporé à nouveau. Le chlorure d'acide obtenu est solubilisé dans 17mL de tétrahydrofurane et est additionné lentement à une solution de 1g de dérivé 2a (4,14 mmoles) et de 0,62mL de triéthylamine (4,43 mmoles) dans 40mL de tétrahydrofurane refroidie à 0°C. Après retour à température ambiante, le milieu est dilué à l'acétate d'éthyle et le mélange est lavé avec une solution aqueuse d'acide chlorhydrique 1N, puis la phase organique est lavée à l'eau jusqu'à neutralité et est ensuite séchée sur MgSO₄. Après filtration et concentration à sec, l'huile obtenue est purifiée par chromatographie flash sur silice (Hexane-AcOEt : 50-50). On isole 1,06g de poudre blanche (rendement : 52%).
Point de fusion : 129°C.
CCM gel de silice 60 F 254 Merck, Hexane-AcOEt : 70-30, Rf = 0,26.
RMN 1H (DMSO) ppm: 9,06 (s, 1H), 8,03 (s, 1H), 6,70 (s, 1H), 4,61 (m, 1H), 3, 32 (m, 2H), 2, 54 (m, 1H), 2,24 (m, 1H), 2,10 (s, 3H), 2,09 (s, 3H), 1,97 (s, 3H), 1,65 (m, 3H), 1,48 (s, 3H), 1, 47 (s, 3H), 1,45 (m, 3H), 1,23 (m, 20H), 0,85 (t, 3H).
HMRS-ESI m/z: 532,81 calculé pour C₃₁H₅₂N₂O₃S; trouvé: 555,35 C₃₁H₅₂N₂NaO₃S (MNa+)
Analyse pour C₃₁H₅₂N₂O₃S, calculé : C, 69,88 ; H, 9,84 ; N, 5,24 ; S, 6,02 ; trouvé : C, 70,23 ; H, 9, 79 ; N, 5,28 ; S, 6,00.
HPLC: 99,95%

### Exemple 2: 1-(2-(dodécylthio)-2-méthylpropanoyl)-N-(4-hydroxy-2,3,5-triméthyl-phényl)-pyrrolidine-2-carboxamide

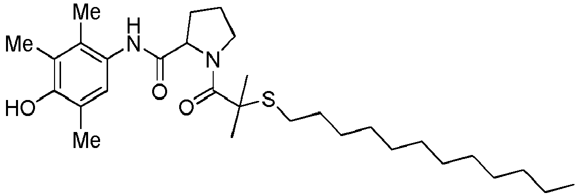

Le composé 2 (solide) est préparé à partir des dérivés la et 2b selon la méthode synthèse 1 dans les conditions opératoires décrites pour l'exemple 1 (rendement : 56%).
Point de fusion : 121°C.
CCM gel de silice 60 F 254 Merck, Hexane-AcOEt : 50-50, Rf = 0,38.
RMN 1H (DMSO) ppm: 9,06 (s, 1H), 7,99 (s, 1H), 6,72 (s, 1H), 4,43 (m, 1H), 4,13 (m, 1H), 3,84 (m, 1H), 2,47 (m, 2H), 2,10 (s, 3H), 2,08 (s, 3H), 1,97 (m, 3H), 1,80 (m, 2H), 1,45 (s, 3H), 1,44 (s, 3H), 1,40 (m, 3H), 1,23 (m, 19H), 0,85 (t, 3H). HMRS-ESI m/z: 518,78 calculé pour C₃₀H₅₀N₂O₃S; trouvé: 541,34 C₃₀H₅₀N₂NaO₃S (MNa+)
Analyse pour C₃₀H₅₀N₂O₃S, calculé : C, 69,45 ; H, 9,71 ; N, 5,40 ; S, 6,18 ; trouvé : C, 69,87 ; H, 9,64 ; N, 5,40 ; S, 6,14.
HPLC: 99,99%

### Exemple 3: (S)-1-(2-(dodécylthio)-2-méthylpropanoyl)-N-(4-hydroxy-2,3,5-triméthyl-phényl)pyrrolidine-2-carboxamide

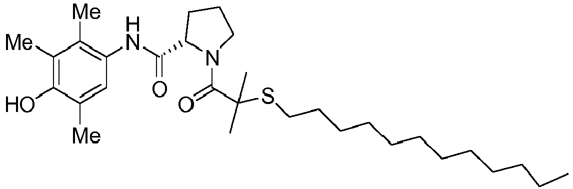

Le composé 3 (solide) est préparé à partir des dérivés 1a et 2c selon la méthode synthèse 1 dans les conditions opératoires décrites pour l'exemple 1 (rendement : 68%).
Point de fusion : 44°C.
CCM gel de silice 60 F 254 Merck, Hexane-AcOEt : 50-50, Rf = 0,56.
α ²⁵_{D}= - 60,5° (EtOH; c=0,25).
RMN 1H (DMSO) ppm: 9,07 (s, 1H), 7,99 (s, 1H), 6,72 (s, 1H), 4,43 (m, 1H), 4,13 (m, 1H), 3,84 (m, 1H), 2,42 (m, 2H), 2,10 (s, 3H), 2,08 (s, 3H), 1,97 (m, 3H), 1,80 (m, 2H), 1,45 (s, 3H), 1,44 (s, 3H), 1,40 (m, 3H), 1,23 (m, 19H), 0,85 (t, 3H).
MS (+ESI) m/z 519 (MH+)
UPLC: 98,96%

### Exemple 4: (S)-1-(2-(décylthio)-2-méthylpropanoyl)-N-(4-hydroxy-2,3,5-triméthyl-phényl)-pyrrolidine-2-carboxamide

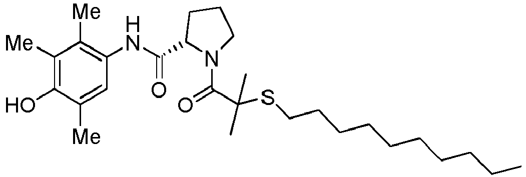

Le composé 4 (huile) est préparé à partir des dérivés 1b et 2c selon la méthode synthèse 1 dans les conditions opératoires décrites pour l'exemple 1 (rendement : 84%).
CCM gel de silice 60 F 254 Merck, Ether de pétrole-AcOEt : 70-30, Rf = 0,35.
RMN 1H (DMSO) ppm: 9,07 (s, 1H), 8,00 (s, 1H), 6,72 (s, 1H), 4,43 (m, 1H), 4,43 (m, 1H), 4,14 (m, 1H), 3,85 (m, 1H), 2,42 (m, 2H), 2,10 (s, 3H), 2,08 (s, 3H), 1,98 (m, 3H), 1,81 (m, 2H), 1,46 (s, 3H), 1,44 (s, 3H), 1,40 (m, 2H), 1,23 (m, 15H), 0,85 (t, 3H).
HMRS-ESI m/z: 490,32 calculé pour C₂₈H₄₆N₂O₃S; trouvé: 513,31 C₂₈H₄₆N₂NaO₃S (MNa+)
Analyse pour C₂₈H₄₆N₂O₃S, calculé : C, 68,53 ; H, 9,45 ; N, 5,71 ; S, 6,53 ; trouvé : C, 68,60 ; H, 9,29 ; N, 5,61 ; S, 6,42.
UPLC: 99,23%

### Exemple 5: (R)-3-(2-(dodécylthio)-2-méthylpropanoyl)-N-(4-hydroxy-2,3,5-triméthyl-phényl)thiazolidine-4-carboxamide

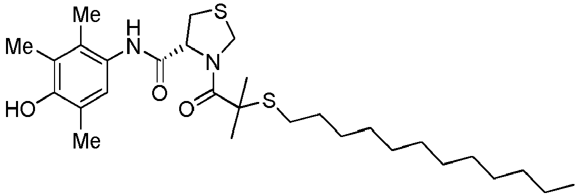

Le composé 5 (huile) est préparé à partir des dérivés 1a et 2d selon la méthode synthèse 1 dans les conditions opératoires décrites pour l'exemple 1 (rendement : 60%).
CCM gel de silice 60 F 254 Merck, Ether de pétrole -AcOEt : 50-50, Rf = 0, 20.
α ²⁵ _{D} = - 100,7° (MeOH)
RMN 1H (DMSO) ppm: 9,28 (s, 1H), 8,04 (s, 1H), 6,73 (s, 1H), 5,5 (m, 1H), 5,1 (m, 1H), 4,58 (m, 1H), 3,50 (m, 1H), 3,09 (m, 1H), 2,50 (m, 2H), 2,10 (s, 3H), 2,08 (s, 3H), 1,98 (m, 3H), 1,48 (s, 3H), 1,46 (s, 3H), 1,44 (m, 2H), 1,23 (m, 18H), 0,85 (t, 3H).
HMRS-ESI m/z: 536,31 calculé pour C₂₉H₄₈N₂O₃S₂; trouvé: 559,29 C₂₉H₄₈N₂NaO₃S₂ (MNa+)
Analyse pour C₂₉H₄₈N₂O₃S₂, calculé : C, 64, 88 ; H, 9, 01 ; N, 5,22 ; S, 11,95 ; trouvé : C, 65,15 ; H, 8,89 ; N, 5,15 ; S, 11,86.
UPLC: 98,93%

### Exemple 6: 2-(dodécylthio)-N-(4-hydroxy-2,3,5-triméthylphényl)-2-(thiophén-3-yl)acétamide

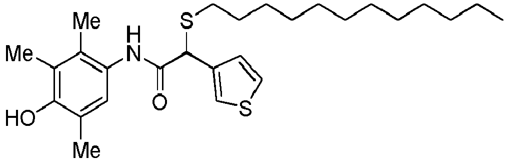

Le composé 6 est préparé selon la méthode de synthèse 2: A une suspension de 772mg de chlorhydrate de 4-amino-2,3,6-triméthylphénol (4,11 mmoles) dans 65mL de dichlorométhane, sous azote à température ambiante, sont ajoutés successivement 0,57mL de triéthylamine (4,11mmoles), puis 1,5g de dérivé 3a et 903mg de dicyclohexylcarbodiimide (4,38 mmoles). Après agitation pendant 8h, le milieu réactionnel est filtré sur büchner, concentré à sec, repris à l'acétate d'éthyle et le mélange est lavé avec une solution aqueuse d'acide chlorhydrique 1N. La phase organique est lavée à l'eau, puis avec une solution aqueuse saturée en NaHCO₃, puis avec solution aqueuse saturée en NaCl, elle est ensuite séchée sur MgSO₄. Après filtration et concentration à sec, l'huile obtenue est purifiée par chromatographie flash sur silice (Ether de pétrole-AcOEt : 80-20). On isole 1,45g de poudre blanc cassé (rendement : 74%).
Point de fusion : 109°C.
CCM gel de silice 60 F 254 Merck, Ether de pétrole-AcOEt : 80-20, Rf = 0,40.
RMN 1H (DMSO) ppm: 9,45 (s, 1H), 8,06 (s, 1H), 7,51 (m, 1H), 7,48 (s, 1H), 7,21 (d, 1H), 6,72 (s, 1H), 4,89 (s, 1H), 2,50 (m, 2H), 2,10 (s, 3H), 2,08 (s, 3H), 1,93 (m, 3H), 1,51 (m, 2H), 1,23 (m, 18H), 0,85 (t, 3H).
HMRS-ESI m/z: 475,76 calculé pour C₂₇H₄₁NO₂S₂; trouvé: 498,24 C₂₇H₄₁NNaO₂S₂ (MNa+)
Analyse pour C₂₇H₄₁NO₂S₂, calculé : C, 68,16 ; H, 8,69 ; N, 2,94 ; S, 13,48 ; trouvé : C, 68,61 ; H, 8,65 ; N, 3,05 ; S, 13,46.
UPLC: 99,75%

### Exemple 7: chlorhydrate du N-(4-amino-2,3,5,6-tétraméthylphényl)-2-(dodécylthio)-2-(thiophén-3-yl)acétamide

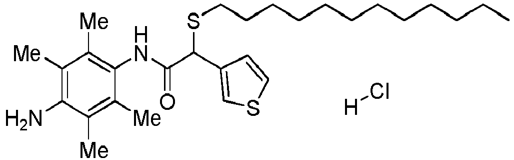

Le composé 7 est préparé à partir de l'intermédiaire 3a et de la 2,3,5,6-tétraméthylbenzène-1,4-diamine selon la méthode synthèse 2 dans les conditions opératoires décrites pour l'exemple 6. Le chlorhydrate est obtenu par salification à l'aide d'une solution 5N d'HCl dans l'isopropanol (1 équivalent) (rendement: 54%).
Point de fusion : 225°C.
CCM gel de silice 60 F 254 Merck, CH₂Cl₂-AcOEt : 95-5, Rf = 0,59. RMN 1H (DMSO) ppm: 9,58 (s, 1H), 7,52 (m, 2H), 7,22 (d, 1H), 4,96 (s, 1H), 3,37 (m, 2H), 2,50 (m, 2H), 2,12 (m, 6H), 2,06 (s, 3H), 1,83 (m, 3H), 1,52 (m, 2H), 1,23 (m, 18H), 0,85 (t, 3H).
HMRS-ESI m/z: 488,81 calculé pour C₂₈H₄₄N₂OS₂; trouvé: 489,29 C₂₈H₄₅N₂OS₂ (MH+)
Analyse pour C₂₈H₄₄N₂OS₂, calculé : C, 64, 03 ; H, 8,64 ; N, 5,33 ; S, 12,21 ; trouvé : C, 64,32 ; H, 8,51 ; N, 5,35 ; S, 12,18.
UPLC: 99,63%

### Exemple 8: chlorhydrate du N-(4-amino-2,3,5,6-tétraméthylphényl)-2-(dodécylthio)-2-(thiophén-2-yl)acétamide

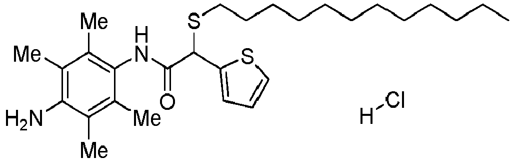

Le composé 8 est préparé à partir de l'intermédiaire 3b et de la 2,3,5,6-tétraméthylbenzène-1,4-diamine selon la méthode synthèse 2 dans les conditions opératoires décrites pour l'exemple 6. Le chlorhydrate est obtenu par salification à l'aide d'une solution 5N d'HCl dans l'isopropanol (1 équivalent)
(rendement: 41%).
Point de fusion : 234°C.
CCM gel de silice 60 F 254 Merck, CH₂Cl₂-AcOEt : 90-10, Rf = 0,40.
RMN 1H (DMSO) ppm: 9,69 (s, 1H), 7,47 (d, 1H), 7,14 (d, 1H), 6,98 (m, 1H) , 5,20 (s, 1H), 3,42 (m, 2H), 2,50 (m, 2H), 2,16 (m, 6H), 2,09 (s, 3H), 1,86 (m, 3H), 1,53 (m, 2H), 1,23 (m, 18H), 0,85 (t, 3H).
HMRS-ESI m/z: 488,81 calculé pour C₂₈H₄₄N₂OS₂; trouvé: 489,29 C₂₈H₄₅N₂OS₂ (MH+)
Analyse pour C₂₈H₄₄N₂OS₂, calculé : C, 64, 03 ; H, 8,64 ; N, 5,33 ; S, 12,21 ; trouvé : C, 64,67 ; H, 8,62 ; N, 5,42 ; S, 12,24.
UPLC: 99,72%

### Exemple 9: Activités biologiques des composés exemplifiés - Inhibition de l'ACAT

Les expérimentations utilisent la méthode de Chautan M. et coll., (Anal. Biochem. 1988) afin de déterminer l'activité ACAT. Les activités sont exprimées en concentrations inhibitrices à 50% (CI₅₀), l'unité est le nM. Deux types de préparations enzymatiques ont été utilisés pour analyser l'activité putative des composés : microsomes hépatiques de rat et cellules humaines intactes HepG2. Les microsomes sont obtenus à partir de fractionnement subcellulaire utilisant une étape d'homogénéisation puis une étape d'ultracentrifugation. Le dosage de l'activité ACAT est réalisé par incubation des microsomes hépatiques ou des cellules avec le composé d'intérêt ainsi que le précurseur radiomarqué. La séparation du produit de la réaction à partir du milieu d'incubation acellulaire ou cellulaire est réalisée par élution sur colonne de silice ; le cholesteryl-oléate formé est ensuite quantifié en radioanalyse.

| Composés | microsomes CI₅₀ (nM) | HepG2 CI₅₀ (nM) |
|---|---|---|
| Exemple 1: | 47 | 47 |
| 1-(2-(dodécylthio)-2-méthylpropanoyl)-N-(4-hydroxy-2,3,5-triméthyl-phényl)pipéridine-2-carboxamide | | |
| Exemple 2: | 81 | 48 |
| 1-(2-(dodécylthio)-2-méthylpropanoyl)-N-(4-hydroxy-2,3,5-triméthyl-phényl)-pyrrolidine-2-carboxamide | | |
| Exemple 3: | 18 | 34 |
| (S)-1-(2-(dodécylthio)-2-méthylpropanoyl)-N-(4-hydroxy-2,3,5-triméthyl-phényl)pyrrolidine-2-carboxamide | | |
| Exemple 4: | 30<CI₅₀<300 | 10<CI₅₀<100 |
| (S)-1-(2-(décylthio)-2-méthylpropanoyl)-N-(4-hydroxy-2,3,5-triméthyl-phényl)-pyrrolidine-2-carboxamide | | |
| Exemple 5: | 34 | 39 |
| (R)-3-(2-(dodécylthio)-2-méthylpropanoyl)-N-(4-hydroxy-2,3,5-triméthyl-phényl)thiazolidine-4-carboxamide | | |
| Exemple 6: | 71 | 27 |
| 2-(dodécylthio)-N-(4-hydroxy-2,3,5-triméthylphényl)-2-(thiophén-3-yl)acétamide | | |
| Exemple 7: | 41 | 93 |
| chlorhydrate du N-(4-amino-2,3,5,6-tétraméthylphényl)-2-(dodécylthio)-2-(thiophén-3-yl)acétamide | | |
| Exemple 8: | 69 | 38 |
| chlorhydrate du N-(4-amino-2,3,5,6-tétraméthylphényl)-2-(dodécylthio)-2-(thiophén-2-yl)acétamide | | |

### Exemple 10: Activités biologique des composés exemplifiés - Inhibition de l'ACAT in vivo

Les rats sont soumis à un régime alimentaire hypercholestérolémique pendant 4 jours. Les animaux sont traités simultanément par voie orale avec les différentes molécules. Le cinquième jour les animaux sont euthanasiés et la variation des taux de cholestérol plasmatique circulant analysée par méthode colorimétrique. Les activités sont exprimées en doses efficaces à 50% (DE₅₀), l'unité est le mg/kg.

| Composés | Etude *in vivo* chez le rat DE₅₀ (mg/kg) |
|---|---|
| Exemple 1: | 0.63 |
| 1-(2-(dodécylthio)-2-méthylpropanoyl)-N-(4-hydroxy-2,3,5-triméthyl-phényl)pipéridine-2-carboxamide | |
| Exemple 2: | <0.63 |
| 1-(2-(dodécylthio)-2-méthylpropanoyl)-N-(4-hydroxy-2,3,5-triméthyl-phényl)-pyrrolidine-2-carboxamide | |
| Exemple 3: | <0.63 |
| (S)-1-(2-(dodécylthio)-2-méthylpropanoyl)-N-(4-hydroxy-2,3,5-triméthyl-phényl)pyrrolidine-2-carboxamide | |
| Exemple 4: | 0.63 |
| (S)-1-(2-(décylthio)-2-méthylpropanoyl)-N-(4-hydroxy-2,3,5-triméthyl-phényl)-pyrrolidine-2-carboxamide | |
| Exemple 5: | <0.63 |
| (R)-3-(2-(dodécylthio)-2-méthylpropanoyl)-N-(4-hydroxy-2,3,5-triméthyl-phényl)thiazolidine-4-carboxamide | |
| Exemple 6: | 0.17 |
| 2-(dodécylthio)-N-(4-hydroxy-2,3,5-triméthylphényl)-2-(thiophén-3-yl)acétamide | |
| Exemple 7: | 0.16 |
| chlorhydrate du N-(4-amino-2,3,5,6-tétraméthylphényl)-2-(dodécylthio)-2-(thiophén-3-yl)acétamide | |
| Exemple 8: | 0.26 |
| chlorhydrate du N-(4-amino-2,3,5,6-tétraméthylphényl)-2-(dodécylthio)-2-(thiophén-2-yl)acétamide | |

## Revendications

1. Composés répondant à la formule générale (I) : 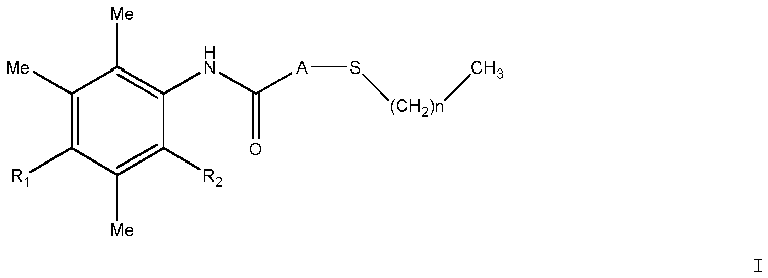 dans laquelle :
- R₁ représente un groupe hydroxyle ou amino
- R₂ représente un hydrogène ou un radical méthyle
- A représente un groupement choisi parmi : 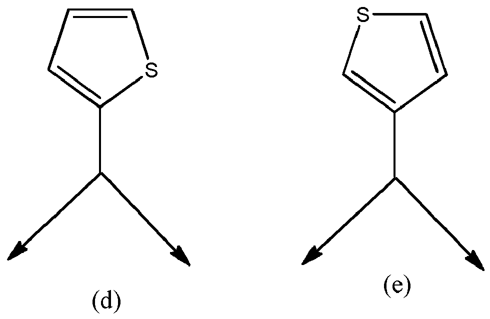
- n représente un nombre entier de 5 à 13,
ainsi que les différents énantiomères et leurs mélanges en toutes proportions, leurs sels pharmaceutiquement acceptables, et leurs solvates.

2. Composés selon la revendication 1 **caractérisés en ce que** A représente le groupement (d), R₁ représente une amine (NH₂), R₂ représente un méthyle, et n représente un nombre entier de 5 à 13.

3. Composés selon la revendication 1 **caractérisés en ce que** A représente le groupement (e), R₁ représente un hydroxyle (OH) ou une amine (NH₂), R₂ représente un hydrogène ou un méthyle, et n représente un nombre entier de 5 à 13.

4. Un composé selon la revendication 1 sélectionné parmi :
2-(dodécylthio)-N-(4-hydroxy-2,3,5-triméthylphényl)-2-(thiophén-3-yl)acétamide
chlorhydrate du N-(4-amino-2,3,5,6-tétraméthylphényl)-2-(dodécylthio)-2-(thiophén-3-yl)acétamide
chlorhydrate du N-(4-amino-2,3,5,6-tétraméthylphényl)-2-(dodécylthio)-2-(thiophén-2-yl)acétamide, ainsi que les différents énantiomères et leurs mélanges en toutes proportions, leurs sels pharmaceutiquement acceptables, et leurs solvates.

5. Procédé de préparation des composés de formule générale (I) selon l'une des revendications 1 à 4 **caractérisé en ce que** l'on condense un intermédiaire de formule générale (IV) 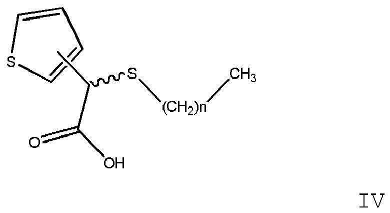 dans lequel n est tel que décrit précédemment dans la formule I et le positionnement de la chaîne soufrée peut être en position 2 ou 3 du thiophène, avec un composé de formule générale V 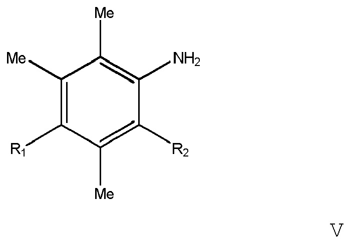 dans lequel R₁ et R₂ représentent les groupements tels que décrits précédemment dans la formule 1.

6. Composés de formule générale I tels que définis selon l'une des revendications 1 à 4 pour leur utilisation en tant que médicament.

7. Composés de formule générale I tels que définis selon l'une des revendications 1 à 4 pour leur utilisation en tant qu'inhibiteur de l'enzyme ACAT.

8. Composés de formule générale I tels que définis selon l'une des revendications 1 à 4 pour leur utilisation en tant que médicament destiné au traitement et/ou à la prévention d'états pathologiques liés à des désordres lipidiques de la peau.

9. Composés de formule générale I tels que définis selon l'une des revendications 1 à 4 pour leur utilisation en tant que médicament destiné au traitement et/ou à la prévention d'acné, de psoriasis, de rosacée, de dermite séborrhéique, de séborrhée, d'eczéma.

10. Composés de formule générale I tels que définis selon l'une des revendications 1 à 4 pour leur utilisation en tant que médicament destiné au traitement et/ou à la prévention de maladies telles que l'obésité, les dyslipidémies, l'hypertriglycéridémie, l'hypercholestérolémie, le syndrome métabolique, l'athérosclérose, la stéatose hépatique, les risques cardiovasculaires.

11. Composés de formule générale I tels que définis selon l'une des revendications 1 à 4 pour leur utilisation en tant que médicament destiné à l'amélioration de la cicatrisation de la peau.

12. Composés de formule générale I tels que définis selon l'une des revendications 1 à 4 pour leur utilisation en tant que médicament destiné à l'amélioration de la cicatrisation cutanée dans le traitement des brûlures de toutes origines, dans le traitement des ulcères de la peau, seul ou en association avec un ou plusieurs composés utile au traitement ou à la prévention des désordres cutanés tels que les rétinoïdes, les antibiotiques, les antibactériens, les anti-androgènes, les antifongiques, les corticoïdes.

13. Composition pharmaceutique **caractérisée en ce qu'**elle contient au moins un composé de formule générale I tel que défini selon l'une des revendications 1 à 4 en association avec tout excipient approprié.

14. Composition pharmaceutique selon la revendication 13, **caractérisée en ce qu'**elle contient au moins un composé de formule générale I tel que défini selon l'une des revendications 1 à 7, en association avec un composé utile dans le traitement ou la prévention des maladies dermatologiques liées aux désordres lipidiques de la peau tel que les rétinoïdes, les antibiotiques, les antibactériens, les anti-androgènes, les antifongiques, les corticoïdes.

15. Composition pharmaceutique selon l'une des revendications 13 et 14, **caractérisée en ce qu'**elle est présentée sous une forme adaptée à l'administration par voie topique.
